# EUROPEAN PATENT SPECIFICATION

(11) **EP 0 502 520 B1**
(45) Date of publication and mention of the grant of the patent: **12.11.1997**
(21) Application number: 92103766.9
(22) Date of filing: 05.03.1992
(51) Int. Cl.: C08K 5/00, C09K 15/04, C07C 233/00

(54) **Stabilizing composition for organic polymers**
Stabilisatorzusammensetzung für organische Polymere
Composition stabilisant pour polymères organiques

(30) Priority: 07.03.1991 IT MI910594
(43) Date of publication of application: 09.09.1992
(73) Proprietor: GREAT LAKES CHEMICAL ITALIA S.r.l., I-20138 Milano (IT)
(72) Inventor: Broussard, Fabio, Dr., I-24060 Brusaporto, Bergamo (IT); Busetto, Carlo, Dr., I-20097 San Donato Milanese, Milan (IT)
(74) Representative: Barz, Peter, Dr.

(56) References cited:
- EP-A- 0 032 586
- EP-A- 0 271 235
- EP-A- 0 309 285
- EP-A- 0 319 771
- US-A- 3 984 460

## Description

The present invention relates to stabilizer compositions having an improved stabilizing effect, obtained by the thermal pretreatment of mixtures of phenolic antioxidants and aminic antioxidants.

The use of sterically hindered phenols in the stabilization of organic materials subject to oxidative deterioration is well known.

In particular, US-A-3,330,859 discloses that sterically hindered phenols of general formula 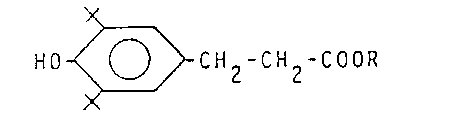 where R is an alkyl radical having from 6 to 20 carbon atoms, are antioxidants for a wide range of polymeric materials, among others the (co)polymers of vinyl chloride, polyolefins such as polyethylene, polypropylene and polybutene, polyurethanes, polyalcohols, polyesters etc., Other materials to which phenolic antioxidants can be added are lubricating oils of the polyester type (for example pentaerythritol tetracapronate), vegetable and mineral oils.

It is also well-known that secondary aromatic amines of general formula 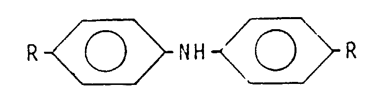 (see US-A-2,776,994) and, among these, preferably those where R = C₈H₁₇; heterocyclic amines of general formula 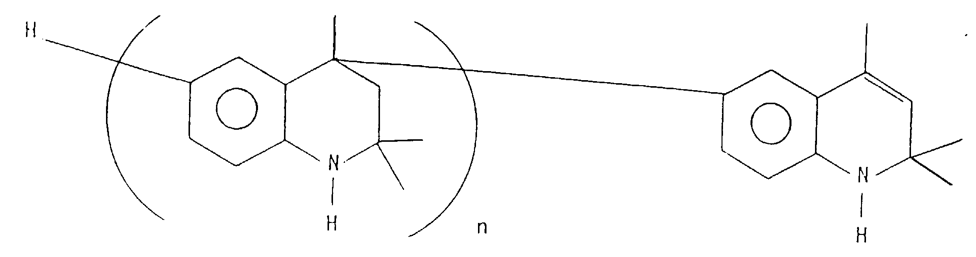 deriving from the condensation of aniline and acetone (J. Voigt, "Die Stabilisierung der Kunststoffe gegen Licht und Wärme" (1986); page 313); the condensation products of diphenylamine and acetone (ibid., page 292), basically composed of 5,5-dimethylacridane; and N,N'-disubstituted para-phenylene diamine (ibid., pages 282-284), are excellent antioxidants for vulcanized and non-vulcanized rubbers, polythenes, polycaprolactams, polyurethanes, etc.,

These amines actually show an antioxidant activity which is even higher than that of the esters of the above-mentioned hydroxyphenyl propanoic acid.

However, because of the colour of the aminic antioxidants (from yellow to brown), the use thereof is limited to the stabilization of polymers whose colour is not particularly important. When the two types of antioxidants, phenolic and aminic antioxidants, are used together, their protective effect on the polymeric substrate is either the same or lower than that of the aminic antioxidant alone.

It has now unexpectedly been found that the same mixtures of antioxidants (phenolic and aminic), if subjected to heating at suitable temperatures, have a protective activity with respect to the polymeric substrates into which they have been incorporated which usually is from 2 to 5 times higher than that of the corresponding, above-mentioned (non-heat-treated) mixtures of the art.

The present invention, therefore, provides stabilizing compositions obtained by thermally treating mixtures of antioxidants containing (a) at least one phenolic antioxidant selected from compounds of general formula (I): 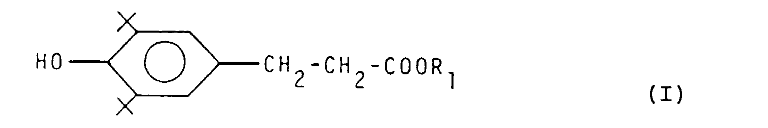 wherein R₁ is a hydrocarbon group having from 4 to 20 (e.g. 8 to 18) carbon atoms; and (b) at least one aminic antioxidant selected from at least one of
(i) compounds of general formula (II): 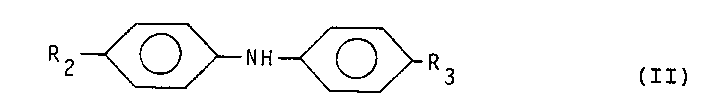 wherein R₂ is hydrogen or a (preferably aliphatic) hydrocarbon group having from 4 to 12 (e.g. 6 to 10) carbon atoms and R₃ is a (preferably aliphatic) hydrocarbon group containing from 1 to 12 (e.g. 3 to 8) carbon atoms;
(ii) the condensation products of aniline and acetone, mainly composed of compounds of general formula (III): 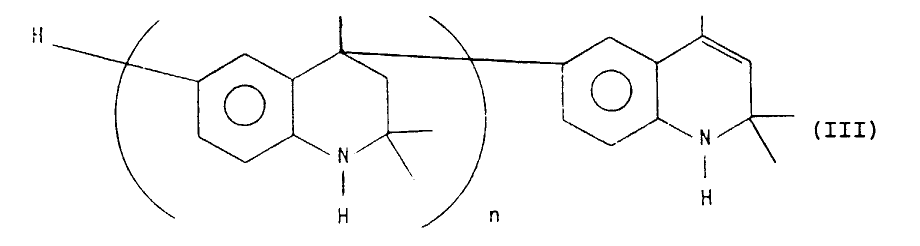 where n is an integer of from 1 to 10;
(iii) the condensation products of diphenylamine and acetone,mainly composed of the compound of formula (IV): 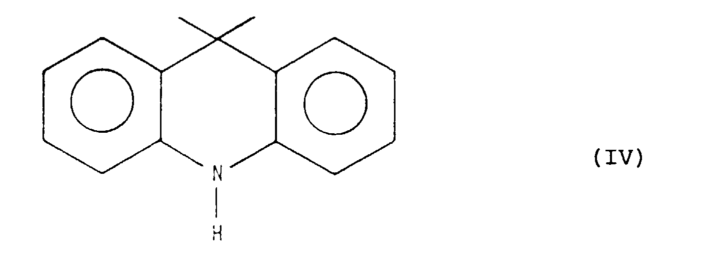 and
(iv) compounds of general formula (V): 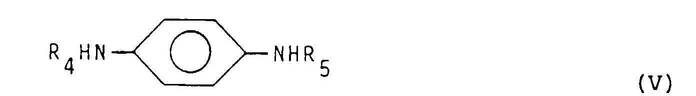 wherein R₄ is an alkyl group containing from 1 to 6 carbon atoms and R₅ is a (preferably C₁-C₁₂, particularly C₁-C₆) alkyl or phenyl group,
wherein the thermal treatment mentioned above comprises the heating of the mixtures at a temperature of from 50 to 150°C, for a period of from 0.3 to 6 hours.

The mixtures are preferably treated at temperatures of from 100 to 110°C for about 3 hours.

The sterically hindered phenolic stabilizer (a) preferably comprises a mixture of the C₁₄-C₁₅ esters of 3-(4-hydroxy-3,5-di-tert-butylphenyl) propanoic acid (formula (I) wherein R₁ is a C₁₄-C₁₅ hydrocarbon group).

As R₁ is derived from a mixture of C₁₄-C₁₅ alcohols (alkanols), the product is a high boiling, light yellow liquid (usually pure esters of the acid are produced in solid form at room temperature).

The effectiveness of the stabilizing mixture obtained by prolonged heat treatment of the mixture of phenolic antioxidant(s) and the aminic antioxidant(s) (II) and/or (III) and/or (IV) and/or (V) was demonstrated on an unstabilized polyol of Dow Chemical.

In order to verify the stabilizing activity of the compositions of the present invention, and in particular to demonstrate the synergetic effect of the thermally treated antioxidant mixtures in preventing the absorption of oxygen by the polymeric system based on a polyol, a differential thermal analysis was carried out to determine the time necessary to induce the oxidation reaction of the system.

The stabilizer was added at levels of 750 to 1500 ppm.

The measurement of the induction period of the oxygen absorption was carried out on the polyol at a temperature of 140°C under an oxygen flow. The longer the induction period of the oxygen absorption, the more effective the stabilizing system used.

The improved stabilizing capacity of the thermally treated mixtures could be demonstrated by determining the induction period of the oxygen absorption by the polyol to which the antioxidant mixture, prepared separately by heating at 100-110°C for several hours, had been added, compared with the induction period of the polyol containing the same stabilizers, but which had been added separately to the polyol.

It was surprisingly found that the induction periods of oxygen absorption were more than doubled with respect to those observed in the case of the mixtures which had not been treated thermally, as is shown in the examples and tables below.

The weight ratios of phenolic and aminic anitoxidants in the preparation of the thermally treated mixture are not critical but are suitably selected so as to obtain a final product in the form of a transparent liquid without any insoluble substances at room temperature.

The weight ratios of phenolic to aminic antioxidant preferably range from 10:1 to 10:10.

Particularly preferred mixtures show a weight ratio of phenolic to aminic antioxidants of about 10:3.

The final product preferably is liquid at room temperature.

This is an enormous advantage with respect to the industrial technique used at present in that the stabilization of the polymers generally requires the addition of either a single solid stabilizer or two separate solid stabilizers such as BHT and an alkylated diphenylamine, with the resulting difficulties and the prolonged periods of time necessary for a double operation.

The following examples provide a further illustration of the present invention but do not limit it in any way.

### EXAMPLE 1

### 1.1 Preparation of phenolic antioxidant (AOX Ib)

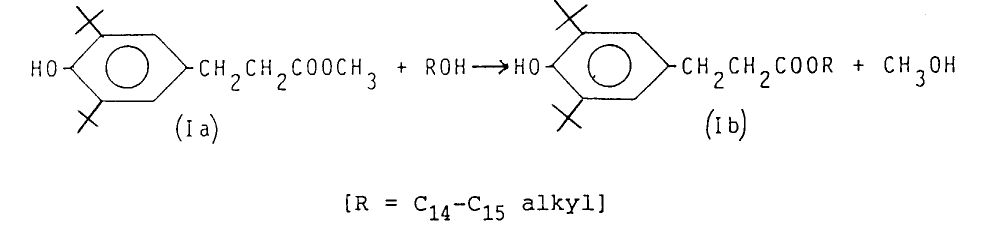

613 g (2.1 moles) of compound (Ia), 436 g (2.0 moles) of a mixture of C₁₄-C₁₅ alcohols and 2 g of a catalyst normally used in transesterification reactions were placed into a 2 litre 4-neck flask equipped with stirrer, thermometer and condenser with collecting flask.

The mixture was subjected to the maximum vacuum of the aspirator (20-30 mmHg) and was heated above the melting point to a temperature of 150-165°C. There was a considerable release of methanol which could be controlled by reducing the amount of heat provided to the system. After two hours the formation of methanol decreased and the temperature was slowly raised to 170°C and kept at said value for 5 hours.

Thereafter the mixture was cooled and the vacuum was increased to a few mmHg by means of a mechanical pump, whereby 40 g of excess compound (Ia) were distilled off along with a small amount of unreacted alcohols.

The final conditions were: 210°C in the boiler, 160°C in the head, vacuum 0.5 mmHg.

The residue in the boiler was cooled to 60°C, atmospheric pressure was re-established and the product (AOX Ib) was discharged.

940 g of a transparent, pale yellow, liquid product, having the following characteristics, were obtained:
- density at 25°C: 0.937 g/cm³
- Höppler viscosity at 30°C: about 220 cps
- acid number: 0.5
- volatile products (2 hours at 105°C): 0.5% b.w.

### 1.2 Preparation of a 10:3 mixture of AOX Ib and aminic antioxidant (2,2,4-trimethyl-1,2-dihydroquinoline homopolymer)

100 g of AOX Ib were placed in a reactor and heated to 100°C. 30 g of chips of aminic antioxidant (hereafter referred to as ANOX HB) were added, in small portions, under stirring.

The dissolution of the ANOX HB in the ester was not immediate and required several minutes. It was difficult to determine the duration of the dissolution because the mass became extremely dark as the ANOX HB dissolved. The mixture was kept at a temperature of 100°C under stirring for 3 hours.

The mass was then cooled to 50°C, transferred to a container and left to cool to room temperature. 130 g of an extremely viscous, transparent, brown, liquid product were obtained.

### 1.3 Test of the stabilizing activity of the mixture prepared under 1.2 above by means of differential thermal analysis

The oxidation kinetics of a polyol, stabilized with mixtures of different additives, were determined by means of differential thermal analysis at 140°C.

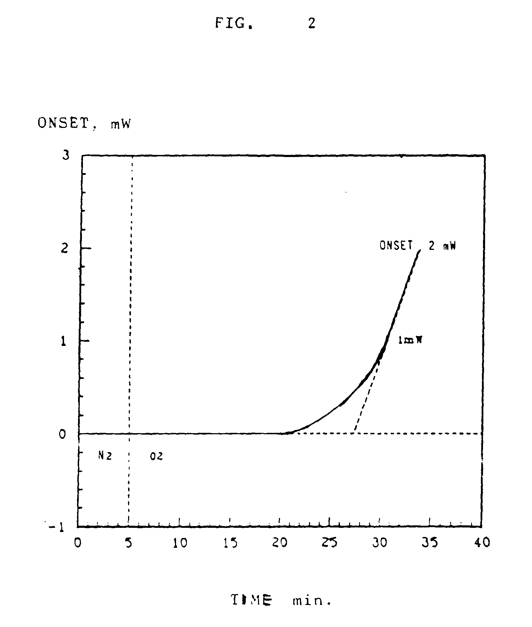

The analyzed systems were brought, under nitrogen, to the analysis temperature inside the measuring capsule within 5 minutes. With an oxygen flow (7 ml/h), the oxidation kinetics were determined in relation to the development of heat during the process. The induction time was calculated at the point of contact of the tangent vector to the oxidation curve with the time axis at 1 mW as shown in Figure 2 which indicates the amount of heat developed (expressed as mW; axis of ordinates) as a function of time.

The addition of the polyol was effected by stirring until the products used had dissolved completely.

An experimental product supplied by Dow Chemical, without stabilizers, was used as the polyol.

Table 1 shows the induction times (in min.) of oxygen absorption in the thermo-oxidation of the polyol at 140°C.

**TABLE 1 -**

| THERMO-OXIDATION OF POLYOLS AT 140°C | | |
|---|---|---|
| Antioxidant | Induction time | % of additive |
| polyol as such | 7.8 min. | ------ |
| AOX Ib | 8.0 min. | 750 ppm |
| ANOX HB | 13.5 min. | 750 ppm |
| thermally treated mixture of AOX Ib and ANOX HB | 32.1 min. | 750 ppm |

### EXAMPLE 2

The product Ib described in example 1 was used as phenolic antioxidant and the condensation product of diphenylamine and acetone (tradename ANOX GAMMA; Bozzetto Rubber Chemicals) was used as aminic antioxidant.

Following the same procedure as in example 1, mixtures of AOX Ib/ANOX GAMMA were prepared in various weight ratios and heated to 110°C for 3 hours.

The synergetic effect of the thermally treated mixtures was demonstrated by means of the following procedure.

Samples of the polyol were prepared and AOX Ib and ANOX GAMMA were mixed directly (and separately) with the polyol in different ratios, the total concentration of the stabilizing components being 750 ppm.

Samples of the polyol to which the thermally treated mixture of AOX Ib and ANOX GAMMA was added in a concentration of 750 ppm were also prepared.

The induction times observed with the various samples are shown in Table 2a (the additives were mixed directly with the polyol without any thermal pretreatment) and in Table 2b (various mixtures of AOX Ib and ANOX GAMMA were added after thermal pretreatment).

**TABLE 2a -**

| THERMO-OXIDATION OF POLYOLS AT 140°C | | | |
|---|---|---|---|
| ANOX GAMMA % | AOX Ib % | ppm in polyol | induction time (minutes) |
| 0 | 100 | 750 | 7.8 |
| 20 | 80 | 750 | 10.6 |
| 50 | 50 | 750 | 13.5 |
| 87.5 | 12.5 | 750 | 19.0 |
| 100 | 0 | 750 | 20.1 |

(The additives were mixed directly with the polyol, without any thermal pre-treatment).

**TABLE 2b -**

| THERMO-OXIDATION OF POLYOLS AT 140°C | | | |
|---|---|---|---|
| ANOX GAMMA % | AOX Ib % | ppm in polyol | induction time (minutes) |
| 15 | 85 | 750 | 24.8 |
| 33 | 67 | 750 | 37.5 |
| 37 | 63 | 750 | 45.0 |
| 50 | 50 | 750 | 54.9 |

(AOX Ib and ANOX GAMMA were mixed and heated for 3 hours at 110°C; concentrations of ANOX GAMMA higher than 50% were not studied as in this case the resulting product was no longer liquid at room temperature.)

It can be noted that there is no synergetic effect of the two stabilizers when they are mixed directly and separately with the polyol and that the stabilizing effect is simply the sum of the activities of the two components, in relation to their weight ratios (Table 2a).

However, in the case of the polyol to which the thermally treated mixture had been added (Table 2b), there was a considerable increase in the stabilizing activity (Table 2b).

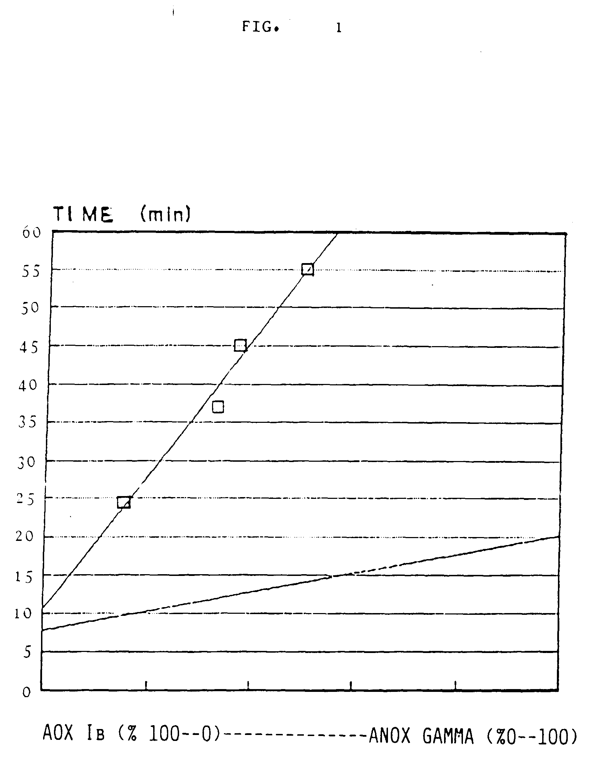

This is also clearly evident from Figure 1 which shows the trends of the induction time in relation to the ratio AOX Ib/ANOX GAMMA in the form of the thermally treated (□) and untreated (---) mixtures, respectively.

### EXAMPLE 3

The product Ib described in example 1 was used as phenolic antioxidant and diphenylamine dioctylate (tradename ANOX NS, Bozzetto Rubber Chemicals) was used as aminic antioxidant.

Using the same procedure as in example 1, mixtures of AOX Ib and ANOX NS were prepared at 110°C with different treatment times (1 hour, 3 hours and 6 hours). The induction times observed in the tests carried out with the polyol are shown in Table 3.

The mixtures were used in concentrations of 1250 ppm in the polyol.

The best results were obtained with a 3 hour heat treatment of the mixture.

**TABLE 3 -**

| THERMO-OXIDATION OF POLYOLS AT 140°C | | | | |
|---|---|---|---|---|
| ANOX-NS parts by weight | AOX Ib parts by weight | ppm in polyol | treatment time (hrs) | induction time (min.) |
| 3 | 10 | 1250 | 1.0 | 34.3 |
| 3 | 10 | 1250 | 3.0 | 54.7 |
| 3 | 10 | 1250 | 6.0 | 15.8 |

(Stabilizing effect of the mixture of ANOX NS and AOX Ib after different treatment times at 110°C).

### EXAMPLE 4

Mixtures of AOX Ib (10 parts by weight) and ANOX GAMMA (3 parts by weight) were prepared as described in example 2.

The mixtures were heated for 1 hour, 1.5 hours, 3 hours and 6 hours, resp., at 100-110°C.

The measurements were carried out with the polyol to which 900 ppm of mixture had been added.

The results obtained are shown in Table 4. Also in this case there is an improved effect due to the prolonged interaction time under heating.

**TABLE 4 -**

| THERMO-OXIDATION OF POLYOLS AT 140°C | | | | |
|---|---|---|---|---|
| ANOX GAMMA parts by weight | AOX Ib parts by weight | ppm in polyol | treatment time (hrs) | induction time (min.) |
| 3 | 10 | 900 | 1.0 | 11.7 |
| 3 | 10 | 900 | 1.5 | 13.0 |
| 3 | 10 | 900 | 3.0 | 26.0 |
| 3 | 10 | 900 | 6.0 | 25.4 |

(Stabilizing effect of the mixture of ANOX GAMMA and AOX Ib after different treatment times at 110°C).

## Claims

1. Stabilizing compositions, comprising mixtures which have been heated at 50 to 150°C for 0.3 to 6 hours and have been prepared from (a) at least one phenolic antioxidant of general formula (I): 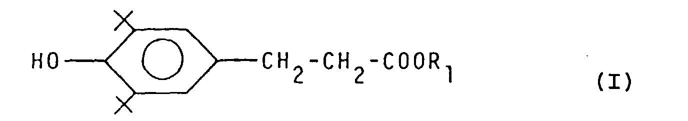 wherein R₁ is a linear or branched C₄-C₂₀ hydrocarbon group; and (b) at least one aminic antioxidant selected from one or more of
(i) compounds of general formula (II): 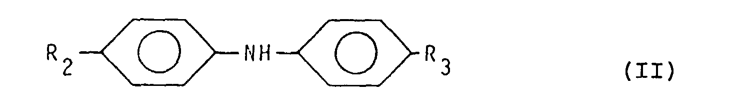 wherein R₂ is hydrogen or a C₄-C₁₂ hydrocarbon group and R₃ is a C₁-C₁₂ hydrocarbon group;
(ii) condensation products of aniline and acetone mainly containing compounds of general formula (III): 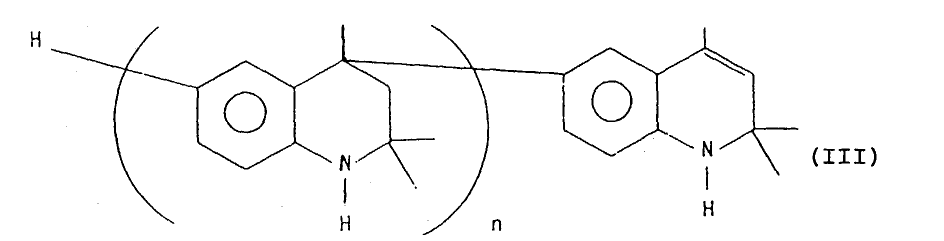 wherein n is an integer of from 1 to 10;
(iii)condensation products of diphenylamine and acetone mainly containing the compound of formula (IV): 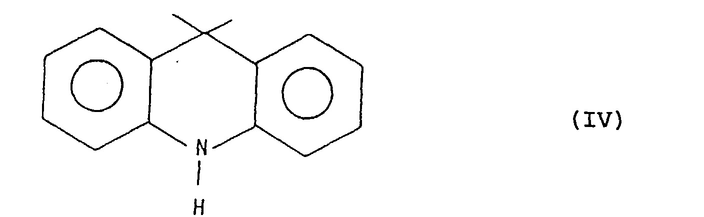 and
(iv) compounds of general formula (V): 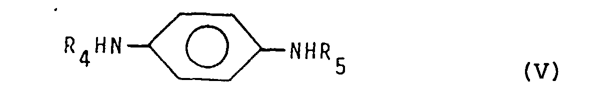 wherein R₄ is C₁-C₆ alkyl and R₅ is an alkyl or phenyl group.

2. Compositions according to claim 1, wherein the mixtures have been heated at a temperature of from 100 to 110°C for about 3 hours.

3. Compositions according to any one of claims 1 and 2, wherein the phenolic antioxidant (a) comprises one or more esters obtained by esterifying 3-(4'-hydroxy-3',5'-di-tert-butyl-phenyl)-propanoic acid with a mixture of alcohols containing 14 to 15 carbon atoms.

4. Compositions according to any one of the preceding claims wherein the weight ratio (a):(b) varies from 10:1 to 10:10 and particularly is about 10:3.

5. Polymeric compositions, comprising the stabilizing compositions of any one of claims 1 to 4.

6. Polymeric compositions according to claim 5, wherein the polymer is selected from polyolefins, vinyl chloride (co)polymers, polyurethanes, polyalcohols, polyesters, polyamides, vulcanized and non-vulcanized rubbers and mixtures of said polymers.

7. Polymeric compositions according to any one of claims 5 and 6 wherein the stabilizing composition is present in amounts of from 100 to 10000 ppm, particularly 500 to 5000 ppm.

8. Molded articles made from the polymeric compositions of any one of claims 5 to 7.

## Patentansprüche

1. Stabilisierende Zusammensetzungen, umfassend Mischungen, die 0,3 bis 6 Stunden bei 50 bis 150°C erwärmt wurden und hergestellt wurden aus (a) mindestens einem phenolischen Antioxidationsmittel der allgemeinen Formel (I): 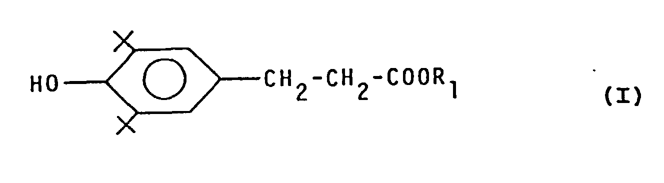 worin R₁ eine lineare oder verzweigte C₄-C₂₀-Kohlenwasserstoffgruppe darstellt; und (b) mindestens einem aminischen Antioxidationsmittel, das ausgewählt ist aus einem oder mehreren von
(i) Verbindungen der allgemeinen Formel (II): 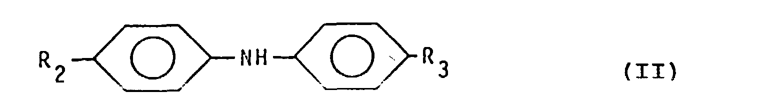 worin R₂ Wasserstoff oder eine C₄-C₁₂-Kohlenwasserstoffgruppe darstellt und R₃ eine C₁-C₁₂-Kohlenwasserstoffgruppe bedeutet;
(ii) Kondensationsprodukten von Anilin und Aceton, die hauptsächlich Verbindungen der allgemeinen Formel (III) enthalten: 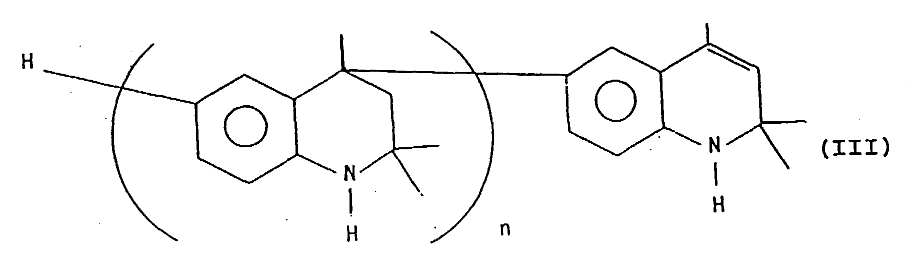 worin n eine ganze Zahl von 1 bis 10 ist;
(iii) Kondensationsprodukten von Diphenylamin und Aceton, die hauptsächlich die Verbindung der Formel (IV) enthalten: 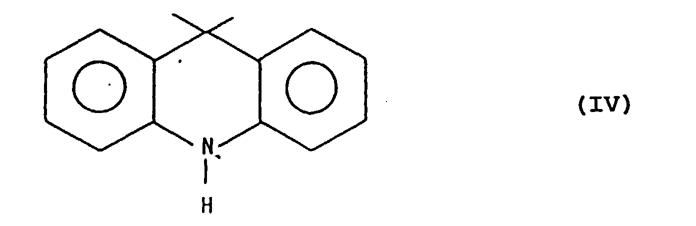 und
(iv) Verbindungen der allgemeinen Formel (V): 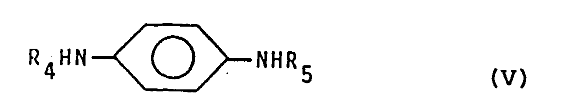 worin R₄ für C₁-C₆-Alkyl steht und R₅ eine Alkyl- oder Phenylgruppe ist.

2. Zusammensetzungen nach Anspruch 1, in welchen die Mischungen etwa 3 Stunden lang auf eine Temperatur von 100 bis 110°C erwärmt wurden.

3. Zusammensetzungen nach irgendeinem der Ansprüche 1 und 2, in welchen das phenolische Antioxidationsmittel (a) einen oder mehrere Ester umfaßt, die durch Veresterung von 3-(4'-Hydroxy-3',5'-di-tert-butylphenyl)propansäure mit einer Mischung von 14 bis 15 Kohlenstoffatome enthaltenden Alkoholen erhalten wurden.

4. Zusammensetzungen nach irgendeinem der vorangehenden Ansprüche, in welchen das Gewichtsverhältnis (a):(b) von 10:1 bis 10:10 variiert und insbesondere etwa 10:3 ist.

5. Polymere Zusammensetzungen, umfassend die stabilisierenden Zusammensetzungen von irgendeinem der Ansprüche 1 bis 4.

6. Polymere Zusammensetzungen nach Anspruch 5, in welchen das Polymer aus Polyolefinen, Vinylchlorid-(Co)polymeren, Polyurethanen, Polyalkoholen, Polyestern, Polyamiden, vulkanisierten und nicht-vulkanisierten Kautschuken und Mischungen dieser Polymere ausgewählt ist.

7. Polymere Zusammensetzungen nach irgendeinem der Ansprüche 5 und 6, in welchen die stabilisierende Zusammensetzung in Mengen von 100 bis 10000 ppm, insbesondere 500 bis 5000 ppm, anwesend ist.

8. Formgegenstände, hergestellt aus den polymeren Zusammensetzungen von irgendeinem der Ansprüche 5 bis 7.

## Revendications

1. Composition stabilisante, comprenant un mélange qui a été traité à 50 à 150°C pendant 0,3 à 6 heures et qui a été préparé à partir de :
(a) au moins un antioxydant phénolique de formule générale (I) : 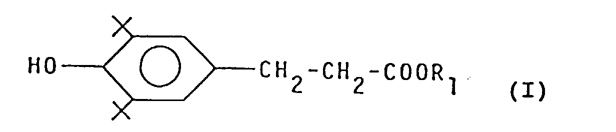 dans laquelle R₁ représente un groupe hydrocarboné linéaire ou ramifié, en C₄ à C₂₀, et
(b) au moins un antioxydant du type amine, constitué d'un ou plusieurs composés choisis parmi :
(i) les composés de formule générale (II) : 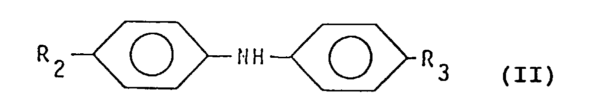 dans laquelle R₂ représente un atome d'hydrogène ou un groupe hydrocarboné en C₄ à C₁₂, et R₃ représente un groupe hydrocarboné en C₁ à C₁₂,
(ii) les produits de condensation d'aniline et d'acétone, contenant principalement des composés de formule générale (III) : 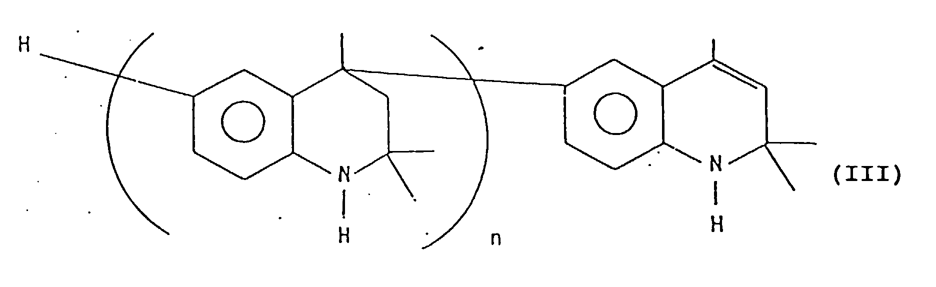 dans laquelle n est un nombre entier ayant une valeur de 1 à 10,
(iii) les produits de condensation de diphénylamine et d'acétone, contenant principalement le composé de formule (IV) : 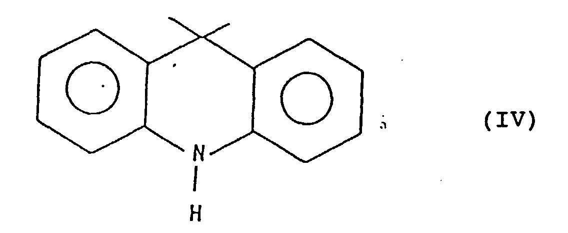 et
(iv) les composés de formule générale (V) : 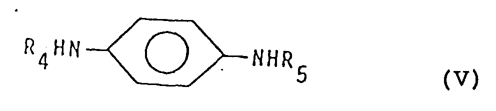 dans laquelle R₄ représente un groupe alkyle en C₁ à C₆ et R₅ représente un groupe alkyle ou phényle.

2. Composition selon la revendication 1, pour laquelle le mélange a été chauffé à une température de 100 à 110°C pendant environ 3 heures.

3. Composition selon la revendication 1 ou 2, dans laquelle l'antioxydant phénolique (a) renferme un ou plusieurs esters obtenus par estérification de l'acide 3-(4'-hydroxy-3',5'-di(tert-butyl) -phényl) propanoïque avec un mélange d'alcools contenant 14 à 15 atomes de carbone.

4. Composition selon l'une quelconque des revendications précédentes, dans laquelle le rapport en poids (a):(b) a une valeur de 10:1 à 10:10 et, en particulier, est égal à environ 10:3.

5. Composition polymère, renfermant la composition stabilisante selon l'une quelconque des revendications 1 à 4.

6. Composition polymère selon la revendication 5, dans laquelle le polymère est un polymère choisi parmi les polyoléfines, les polymères ou copolymères de chlorure de vinyle, les polyuréthanes, les polyalcools, les polyesters, les polyamides, les caoutchou vulcanisés ou non vulcanisés et les mélanges de tels polymères.

7. Composition polymère selon la revendication 5 ou 6, dans laquelle la composition stabilisante est présente à raison de 100 à 10.000 ppm, en particulier à raison de 500 à 5000 ppm.

8. Article moulé obtenu à partir de la composition polymère selon l'une quelconque des revendications 5 à 7.
